# FASCICULE DE BREVET EUROPEEN

(11) **EP 1 366 746 B1**
(45) Date de publication et mention de la délivrance du brevet: **14.04.2010**
(21) Numéro de dépôt: 03291168.7
(22) Date de dépôt: 20.05.2003
(51) Int. Cl.: A61Q 5/02, A61K 8/90, A61Q 5/12

(54) **Shampooing contenant au moins une silicone et au moins un copolymère linéaire séquencé amphiphile, anionique ou non-ionique**
Haarreinigungsmittel, enthaltend mindestens ein Silikon und mindestens ein anionisches oder nicht-ionisches lineares Blockcopolymer
Shampoo contaning at least one silicone and at least one anionic or nonionic amphiphilic linear block copolymer

(30) Priorité: 31.05.2002 FR 0206732
(43) Date de publication de la demande: 03.12.2003
(73) Titulaire: L'Oréal, 75008 Paris (FR)
(72) Inventeur: Dubief, Claude, 78150 Le Chesnay (FR); Restle, Serge, 95390 Saint Prix (FR); Giroud, Franck, 92110 Clichy (FR)
(74) Mandataire: Casalonga, Axel

(56) Documents cités:
- EP-A- 0 463 780
- WO-A-00/21494
- WO-A-99/38476
- WO-A-99/62493
- FR-A- 2 758 262
- FR-A- 2 798 852
- US-A- 5 114 706
- US-A- 5 344 643
- US-A- 5 656 257
- US-A- 5 968 493
- US-A- 6 150 313

## Description

La présente invention concerne des compositions de lavage des fibres kératiniques contenant, dans une base pour shampooings, au moins un copolymère amphiphile linéaire séquencé, anionique ou non-ionique, comprenant au moins un bloc hydrophobe et au moins un bloc hydrophile, et au moins une silicone non-volatile.

Les silicones non-volatiles sont connues depuis longtemps dans le domaine cosmétique, et en particulier dans le domaine capillaire, pour leurs propriétés de conditionnement. En effet, elles facilitent le démêlage des cheveux et leur confèrent un aspect lisse et brillant.
Ces performances conditionnantes des silicones, satisfaisantes sur des cheveux normaux, le sont toutefois nettement moins sur des cheveux sensibilisés par des traitements susceptibles de dégrader la structure des cheveux tels que la coloration ou la décoloration oxydantes et la déformation permanente. Or, ce sont justement les cheveux abîmés par ces traitements qui présentent des problèmes de démêlage et des défauts cosmétiques qui nécessiteraient un effet conditionnant.

Une approche pour résoudre ce problème a consisté à associer les silicones à des polymères cationiques. Il s'est avéré qu'une telle association facilite en effet le démêlage et améliore la douceur des cheveux abîmés, mais ceux-ci deviennent alors trop lisses et fuyants et n'ont plus ni corps ni maintien.
Par ailleurs, l'utilisation de copolymères blocs siliconés, de polyuréthannes à blocs polyester ou polyéther ou de copolymères séquencés à blocs poly(oxyde d'éthylène) et poly(oxyde de propylène), seuls ou en association avec des silicones, s'est avérée être une solution encore moins satisfaisante que celles décrites ci-dessus.

Il est connu des documents FR 2 798 852, US 5,968,493, WO 00/21494, WO 99/38476, US 5,344,643, US 5,114,706 et EP 0 463 780 d'utiliser l'association d'au moins un tensio-actif, d'au moins un polymère et d'au moins un dérivé siliconé pour préparer des compositions pour shampooing.

La demanderesse a constaté avec surprise que l'utilisation d'un groupe de copolymères linéaires séquencés amphiphiles particuliers en association avec au moins une silicone, dans une base particulière pour shampooings, permettait de résoudre les problèmes de l'art antérieur. Les shampooings mis au point par la demanderesse facilitent le démêlage à l'état mouillé et à l'état sec des cheveux et donnent d'excellents résultats cosmétiques, c'est-à-dire confèrent aux cheveux un aspect brillant et un toucher soyeux tout en leur donnant du corps et du maintien, et ceci aussi bien sur des cheveux naturels que sur des cheveux moyennement ou fortement abîmés.

La présente invention a par conséquent pour objet une composition de lavage des matières kératiniques, de préférence des fibres kératiniques, et en particulier des cheveux, contenant, dans un milieu aqueux ou hydroalcoolique cosmétiquement acceptable,
- au moins un copolymère linéaire séquencé amphiphile, anionique ou non-ionique, comprenant au moins un bloc hydrophobe et au moins un bloc hydrophile, à l'exclusion des copolymères séquencés d'oxyde d'éthylène et d'oxyde de propylène, des copolymères séquencés à motifs uréthanne et des copolymères séquencés à motifs siloxane,
- au moins une silicone non-volatile à température ambiante, et
- au moins un agent tensioactif anionique associé à au moins un agent tensioactif non-ionique et/ou au moins un agent tensioactif amphotère.

La présente invention a également pour objet l'utilisation d'une telle composition pour le lavage des fibres kératiniques.

Les copolymères linéaires séquencés, utilisables selon la présente invention, sont des copolymères dits "amphiphiles", à savoir des copolymères comportant au moins un bloc hydrophobe et au moins un bloc hydrophile.
On entend par "bloc hydrophobe" selon la présente invention un bloc comprenant au moins 75 % en moles de monomères insolubles dans l'eau et par "bloc hydrophile", un bloc comprenant au moins 75 % en moles de monomères hydrosolubles.

Les monomères hydrosolubles formant le ou les blocs hydrophiles des copolymères séquencés utilisés dans la présente invention peuvent être de nature anionique ou non-ionique et peuvent être utilisés seuls ou sous forme de mélange contenant deux ou plusieurs monomères différents.

On peut citer à titre d'exemples de monomères hydrosolubles anioniques, les acides carboxyliques à insaturation éthylénique, tels que l'acide acrylique, l'acide méthacrylique, l'acide itaconique, l'acide fumarique, l'acide crotonique et l'acide maléique, l'acide 2-acrylamido-2-méthylpropane-sulfonique, l'acide styrènesulfonique, l'acide vinylsulfonique et l'acide vinylphosphonique.
Les monomères hydrosolubles non-ioniques englobent, entre autres, l'acrylamide, les acrylamides N-alkylés en C₁₋₆ ou N,N-dialkylés en C₁₋₃, l'acrylate de polyéthylèneglycol, le méthacrylate de polyéthylèneglycol, le N-vinylacétamide, le N-méthyl-N-vinylacétamide, le N-vinylformamide, le N-méthyl-N-vinylformamide, les N-vinyllactames comportant un groupe cyclique de 4 à 9 atomes de carbone, l'alcool vinylique (copolymérisé sous forme d'acétate de vinyle puis hydrolysé), l'oxyde d'éthylène, l'acrylate d'hydroxyéthyle, l'acrylate d'hydroxypropyle, le méthacrylate d'hydroxyéthyle et le méthacrylate d'hydroxypropyle.

Les monomères insolubles dans l'eau formant le ou les blocs hydrophobes des copolymères séquencés sont choisis de préférence parmi les monomères vinylaromatiques tels que le styrène et ses dérivés alkylés comme le 4-butylstyrène, l'α-méthylstyrène et le vinyltoluène, les diènes tels que le butadiène et le 1,3-hexadiène, et les dérivés alkylés des diènes tels que l'isoprène et le diméthylbutadiène, le chloroprène, les acrylates d'alkyle en C_{1- 10}, d'aryle en C₆₋₁₀ ou d'aralkyle en C_{6- 10} et les méthacrylates d'alkyle en C_{1-10,} d'aryle en C₆₋₁₀ ou d'aralkyle en C_{6- 10,} comme par exemple les (meth)acrylates de méthyle, d'éthyle, de n-butyle, de 2-éthylhexyle, de tert-butyle, d'isobornyle, de phényle ou de benzyle, l'acétate de vinyle, les vinyléthers de formule CH₂=CH-O-R et les allyléthers de formule CH₂=CH-CH₂-O-R où R représente un groupe alkyle en C_{1- 6,} l'acrylonitrile, le chlorure de vinyle, le chlorure de vinylidène, la caprolactone, l'éthylène, le propylène, les monomères vinyliques fluorés ou à chaîne perfluorée, tels que les acrylates et méthacrylates de fluoroalkyle ou les α-fluoroacrylates d'alkyle.

Comme indiqué ci-dessus à propos de la définition des blocs hydrophobe(s) et hydrophile(s) des copolymères séquencés amphiphiles, les monomères hydrosolubles et les monomères insolubles dans l'eau représentent au moins 75 % en moles respectivement des blocs hydrophobes et hydrophiles. Autrement dit, le ou les blocs hydrophobes peuvent comprendre jusqu'à 25 % en moles d'un ou de plusieurs monomères hydrosolubles. Cette proportion est de préférence au plus égale 10 % en moles et, idéalement, inférieure ou égale à 5 % en moles.
De manière analogue, le ou les blocs hydrophiles peuvent comprendre jusqu'à 25 % en moles, de préférence jusqu'à 10 % en moles, et idéalement jusqu'à 5 % en moles, d'un ou de plusieurs monomères insolubles dans l'eau.
Les copolymères linéaires séquencés utilisés englobent bien entendu également ceux dans lesquels les blocs hydrophiles et les blocs hydrophobes sont constitués exclusivement respectivement de monomères hydrosolubles et de monomères insolubles dans l'eau. Ces blocs peuvent être des blocs homopolymères ou des blocs copolymères renfermant deux ou plus de deux monomères différents du même type.

La masse moléculaire moyenne en nombre de chaque bloc, qu'il soit hydrophobe ou hydrophile, copolymère ou homopolymère, est de préférence comprise entre 500 et 100 000, en particulier entre 500 et 50 000, avec un indice de polydispersité (M_{w}/Mₙ) compris entre 1,01 et 3,0, de préférence entre 1,1 et 2,5.

Les polymères blocs de l'invention peuvent être préparés par les procédés de synthèse classiquement utilisés pour obtenir des polymères blocs. On peut citer par exemple les polymérisations anionique ou cationique, et la polymérisation radicalaire contrôlée (voir "New Method of Polymer Synthesis", Blackie Academic & Professional, Londres, 1995, volume 2, page 1, ou Trends Polym. Sci. 4, page 183 (1996) de C. J. Hawker), qui peut être mise en oeuvre suivant différents procédés comme par exemple la polymérisation radicalaire par transfert d'atomes *(Atom Transfert Radical Polymerization* ou ATRP) (voir JACS, 117, page 5614 (1995), de Matyjasezwski *et al.*), la méthode des radicaux tels que les nitroxydes (Georges et al., Macromolecules, 1993, 26, 2987).
On peut aussi utiliser ces procédés pour obtenir un seul des deux types de blocs du polymère de l'invention, l'autre bloc étant introduit dans le polymère final par l'intermédiaire de l'amorceur utilisé ou bien par réaction de couplage entre les blocs hydrophiles et hydrophobes.

Les compositions de lavage de la présente invention peuvent contenir les copolymères séquencés à l'état dissous ou dispersé, et ces copolymères sont donc de préférence solubles ou dispersibles dans le milieu cosmétique utilisé.
Les copolymères diblocs sont de préférence hydrosolubles.

On entend par "hydrosoluble" des composés (polymères ou monomères) qui, introduits dans l'eau à 25°C, et si besoin neutralisés, à une concentration en poids égale à 0,1 %, permettent l'obtention d'une solution ou d'une suspension macroscopiquement homogène et transparente, c'est-à-dire ayant une valeur de transmittance de la lumière, à une longueur d'onde égale à 500 nm, à travers un échantillon de 1 cm d'épaisseur, d'au moins 70 %, de préférence d'au moins 80 %.

Comme indiqué ci-dessus, les compositions de lavage de la présente invention comprennent - en plus du ou des copolymères séquencés, amphiphiles, anioniques ou non ioniques, décrits ci-dessus - au moins une silicone non-volatile à température ambiante, qui est généralement présente en une concentration comprise entre 0,01 et 20 %, rapportée au poids total de la composition de lavage. On entend par "silicone non-volatile" au sens de la présente invention toute silicone dont la température d'ébullition est supérieure à 245 °C à pression atmosphérique.

Les silicones utilisables conformément à l'invention peuvent être solubles ou insolubles dans l'eau ou la composition. Il s'agit en particulier de polyorganosiloxanes insolubles dans l'eau et dans la composition, et qui se présentent sous forme d'huiles, de cires, de résines et de gommes de silicone.
Les silicones utilisables dans les compositions de lavage peuvent être réticulées ou non-réticulées, organomodifiées ou non.

On entend par silicones insolubles dans l'eau et dans la composition celles ayant une solubilité, mesurée à 25°C, inférieure ou égale à 0,1 % en poids dans l'eau ou dans la composition, c'est-à-dire qu'au-delà de cette concentration, elles ne forment pas de solutions isotropes transparentes.

Les organopolysiloxanes sont définis plus en détail dans l'ouvrage de Walter NOLL "Chemistry and Technology of Silicones" (1968) Académie Press.

Les silicones non-volatiles sont de préférence des polyalkylsiloxanes, des polyarylsiloxanes, des polyalkylarylsiloxanes, des gommes et des résines de silicones, des polyorganosiloxanes modifiés par des groupements organofonctionnels ainsi que leurs mélanges.

Ces silicones sont plus particulièrement choisies parmi les polyalkylsiloxanes parmi lesquels on peut citer principalement les polydiméthylsiloxanes à groupements terminaux triméthylsilyle ayant une viscosité de 5.10-6 à 2,5 m²/s à 25°C et de préférence 1.10⁻⁵ à 1 m²/s.

Parmi ces polyalkylsiloxanes, on peut citer à titre non limitatif les produits commerciaux suivants :
- les huiles SILBIONE® des séries 47 et 70 047 ou les huiles MIRASIL® commercialisées par RHONE POULENC telles que par exemple l'huile 70 047 V 500 000 ;
- les huiles de la série MIRASIL® commercialisées par la société RHONE POULENC ;
- les huiles de la série 200 de la société DOW CORNING telles que plus particulièrement la DC200 de viscosité 60 000 Cst ;
- les huiles VISCASIL® de GENERAL ELECTRIC et certaines huiles des séries SF (SF 96, SF 18) de GENERAL ELECTRIC.

On peut également citer les polydiméthylsiloxanes à groupements terminaux diméthylsilanol (Dimethiconol selon la dénomination CTFA) tels que les huiles de la série 48 de la société RHONE POULENC .

Dans cette classe de polyalkylsiloxanes, on peut également citer les produits commercialisés sous les dénominations "ABIL WAX 9800 et 9801" par la société GOLDSCHMIDT qui sont des polyalkyl (C₁₋C₂₀) siloxanes.

Les polyalkylarylsiloxanes sont particulièrement choisis parmi les polydiméthyl méthylphénylsiloxanes, les polydiméthyl diphénylsiloxanes linéaires et/ou ramifiés de viscosité de 1.10⁻⁵ à 5.10⁻²m²/s à 25°C.

Parmi ces polyalkylarylsiloxanes on peut citer à titre d'exemple les produits commercialisés sous les dénominations suivantes :
- les huiles SILBIONE® de la série 70 641 de RHONE POULENC ;
- les huiles des séries RHODORSIL® 70 633 et 763 de RHONE POULENC;
- l'huile DOW CORNING 556 COSMETIC GRAD FLUID de DOW CORNING;
- les silicones de la série PK de BAYER comme le produit PK20 ;
- les silicones des séries PN, PH de BAYER comme les produits PN1000 et PH1000;
- certaines huiles des séries SF de GENERAL ELECTRIC telles que SF 1023, SF 1154, SF 1250, SF 1265.

Les gommes de silicone utilisables conformément à l'invention sont notamment des polydiorganosiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles polyphénylméthylsiloxanes (PPMS), les isoparaffines, les polyisobutylènes, le chlorure de méthylène, le pentane, le dodécane, le tridécanes ou leurs mélanges.

On peut plus particulièrement citer les produits suivants :
- polydiméthylsiloxane
- les gommes polydiméthylsiloxanes/méthylvinylsiloxane,
- polydiméthylsiloxane/diphénylméthylsiloxane,
- polydiméthylsiloxane/phénylméthylsiloxane,
- polydiméthylsiloxane/diphénylsiloxane/méthylvinylsiloxane.

Des produits plus particulièrement utilisables conformément à l'invention sont des mélanges tels que :
- les mélanges formés à partir d'un polydiméthylsiloxane hydroxylé en bout de chaîne (dénommé diméthiconol selon la nomenclature du dictionnaire CTFA) et d'un poly-diméthylsiloxane cyclique (dénommé cyclométhicone selon la nomenclature du dictionnaire CTFA) tel que le produit Q2 1401 commercialisé par la société DOW CORNING;
- les mélanges formés à partir d'une gomme polydiméthylsiloxane avec une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10-6m²/s. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les unités :
R₂SiO_{2/2,} R₃SiO_{1/2,} RSiO_{3/2} et SiO_{4/2} dans lesquelles R représente un groupement hydrocarboné possédant 1 à 16 atomes de carbone ou un groupement phényle. Parmi ces produits, ceux particulièrement préférés sont ceux dans lesquels R désigne un radical alkyle inférieur en C₁-C₄, plus particulièrement méthyle, ou un radical phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organo-modifiées utilisables conformément à l'invention sont des silicones telles que définies précédemment et comportant, dans leur structure, un ou plusieurs groupements organofonctionnels fixés par l'intermédiaire d'un radical hydrocarboné.

Parmi les silicones organomodifiées, on peut citer les polyorganosiloxanes comportant
- des groupements polyéthylèneoxy et/ou polypropylèneoxy comportant éventuellement des groupements alkyle en C₆-C₂₄ tels que les produits dénommés diméthicone copolyol commercialisé par la société DOW CORNING sous la dénomination DC 1248 ou les huiles SILWET L 722, L 7500, L 77, L 711 de la société UNION CARBIDE et l'alkyl(C₁₂)méthicone-copolyol commercialisée par la société DOW CORNING sous la dénomination Q2 5200 ;
- des groupements aminés substitués ou non comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄;
- des groupements thiols comme les produits commercialisés sous les dénominations "GP 72 A" et "GP 71" de GENESEE ;
- des groupements alcoxylés comme le produit commercialisé sous la dénomination "SILICONE COPOLYMER F-755" par SWS SILICONES et ABIL WAX 2428, 2434 et 2440 par la société GOLDSCHMIDT ;
- des groupements hydroxylés comme les polyorganosiloxanes à fonction hydroxyalkyle décrits dans la demande de brevet français FR-A-85 16334 répondant à la formule (V) : 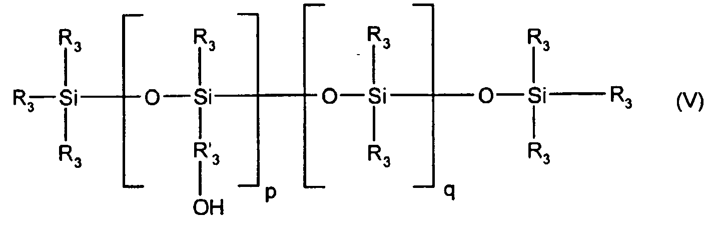 dans laquelle les radicaux R₃ identiques ou différents sont choisis parmi les radicaux méthyle et phényle ; au moins 60 % en mole des radicaux R₃ désignant méthyle ; le radical R'₃ est un chaînon alkylène divalent hydrocarboné en C₂-C₁₈ ; p est compris entre 1 et 30 inclus ; q est compris entre 1 et 150 inclus ;
- des groupements acyloxyalkyle tels que par exemple les polyorganosiloxanes décrits dans la demande de brevet français FR-A-2641185 et répondant à la formule (VI): 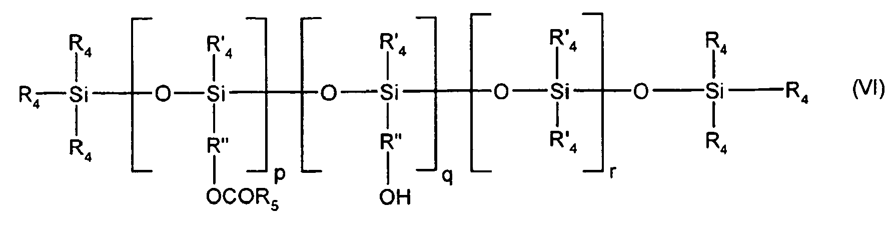 dans laquelle :
   R₄ désigne un groupement méthyle, phényle, -OCOR₅, hydroxyle, un seul des radicaux R₄ par atome de silicium pouvant être OH ;
   chaque R'₄ désigne un groupe méthyle, phényle ; au moins 60 % en proportion molaire de l'ensemble des radicaux R₄ et R'₄ désignant un groupe méthyle ;
   R₅ désigne un groupe alkyle ou alcényle en C₈-C₂₀;
   R" désigne un radical alkylène hydrocarboné divalent, linéaire ou ramifié en C₂-C₁₈;
   r est compris entre 1 et 120 inclus ;
   p est compris entre 1 et 30 ;
   q est égal à 0 ou est inférieur à 0,5 p, p + q étant compris entre 1 et 30 ; les polyorganosiloxanes de formule (VI) peuvent contenir des groupements : 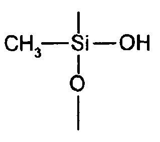 dans des proportions ne dépassant pas 15 % de la somme p + q + r.
   Les composés de formule (VI) peuvent être préparés par estérification de polyorganosiloxanes à fonction hydroxyalkyle de formule (V) ci-dessus.
- des groupements anioniques du type carboxylique comme par exemple dans les produits décrits dans le brevet EP 186 507 de la société CHISSO CORPORATION, ou de type alkylcarboxyliques comme ceux présents dans le produit X-22-3701E de la société SHIN-ETSU ; 2-hydroxyalkyisulfonate ; 2-hydroxyalkylthiosulfate tels que les produits commercialisés par la société GOLDSCHMIDT sous les dénominations "ABIL S201" et "ABIL S255".
- des groupements hydroxyacylamino, comme les polyorganosiloxanes décrits dans la demande EP 342 834. On peut citer par exemple le produit Q2-8413 de la société DOW CORNING.

Selon l'invention, on peut également utiliser des silicones comprenant une portion polysiloxane et une portion constituée d'une chaîne organique non-siliconée, l'une des deux portions constituant la chaîne principale du polymère l'autre étant greffée sur ladite chaîne principale. Ces polymères sont par exemple décrits dans les demandes de brevet EP-A-412 704, EP-A-412 707, EP-A-640 105 et WO 95/00578, EP-A-582 152 et WO 93/23009 et les brevets US 4 693 935, US 4 728 571 et US 4 972 037. Ces polymères sont de préférence anioniques ou non ioniques.
De tels polymères sont par exemple les copolymères susceptibles d'être obtenus par polymérisation radicalaire à partir du mélange de monomères constitué par :
a) 50 à 90% en poids d'acrylate de tertiobutyle ;
b) 0 à 40% en poids d'acide acrylique ;
c) 5 à 40% en poids de macromère siliconé de formule : 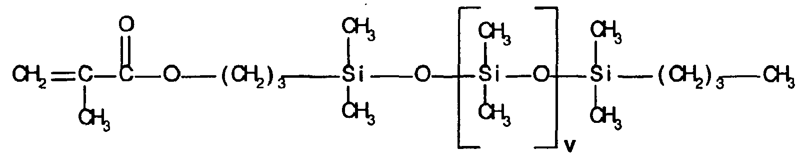
avec v étant un nombre allant de 5 à 700 ; les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

Selon l'invention, toutes les silicones peuvent également être utilisées sous forme d'émulsions.

Les silicones particulièrement préférées conformément à l'invention sont :
- les huiles ou gommes polydiméthylsiloxane,
- les polysiloxanes à groupements aminés tels que les amodiméthicones ou les triméthylsilylamodiméthicones ;

L'association des deux types de polymères essentiels pour la présente invention décrits ci-dessus (silicone + copolymère séquencés), se trouve dans une base pour shampooings spécifique contenant l'association d'au moins un agent tensioactif anionique et d'au moins un agent tensioactif non-ionique et/ou d'au moins un agent tensioactif amphotère.

Les agents tensioactifs anioniques, non-ioniques et amphotères utilisables dans les compositions de lavage de la présente invention sont connus et couramment utilisés dans le domaine cosmétique.

Comme agents tensioactifs anioniques utilisables dans la présente invention, on peut notamment mentionner les sels, en particulier les sels de métaux alcalins tels que les sels de sodium, les sels d'ammonium, les sels d'aminés, les sels d'aminoalcools ou les sels de métaux alcalino-terreux, par exemple, de magnésium, des types suivants : les alkylsulfates, les alkyléthersulfates, les alkylamidoéthersulfates, les alkylarylpolyéthersulfates, les monoglycéride-sulfates ; les alkylsulfonates, les alkylamidesulfonates, les alkylarylsulfonates, les α-oléfine-sulfonates, les paraffine-sulfonates, les alkylsulfosuccinates, les alkyléthersulfosuccinates, les alkylamidesulfosuccinates, les alkylsulfo-acétates, les acylsarcosinates et les acylglutamates, les groupes alkyle et acyle de tous ces composés comportant de 6 à 24 atomes de carbone et le groupe aryle désignant de préférence un groupe phényle ou benzyle.
On peut également utiliser les monoesters d'alkyle en C₆₋₂₄ et d'acides polyglycoside-dicarboxyliques tels que les glucoside-citrates d'alkyle, les polyglycoside-tartrates d'alkyle et les polyglycoside-sulfosuccinates d'alkyle, les alkylsulfosuccinamates, les acyliséthionates et les N-acyltaurates, le groupe alkyle ou acyle de tous ces composés comportant de 12 à 20 atomes de carbone.

Un autre groupe d'agents tensioactifs anioniques utilisables dans les compositions de la présente invention est celui des acyl-lactylates dont le groupe acyle comporte de 8 à 20 atomes de carbone.
En outre, on peut encore citer les acides alkyl-D-galactoside-uroniques et leurs sels ainsi que les acides alkyl(C₆₋₂₄)éther-carboxyliques polyoxyalkylénés, les acides alkyl(C₆₋₂₄)aryl(C₆₋₂₄)éther-carboxyliques polyoxyalkylénés, les acides alkyl(C₆₋₂₄)amidoéther-carboxyliques polyoxyalkylénés et leurs sels, en particulier ceux comportant de 2 à 50 motifs oxyde d'éthylène, et leurs mélanges.

On utilise de préférence les alkylsulfates, les alkyléthersulfates et les alkyléthercarboxylates, et leurs mélanges, en particulier sous forme de sels de métaux alcalins ou alcalino-terreux, d'ammonium, d'aminé ou d'aminoalcool.

Les agents tensioactifs amphotères, utilisables dans la présente invention, peuvent être notamment des dérivés d'aminés aliphatiques secondaires ou tertiaires, dans lesquels le groupe aliphatique est une chaîne linéaire ou ramifiée comportant de 8 à 22 atomes de carbone et contenant au moins un groupe anionique tel que, par exemple, un groupe carboxylate, sulfonate, sulfate, phosphate ou phosphonate. On peut citer en particulier les alkyl(C₈₋₂₀)bétaïnes, les sulfobétaïnes, les alkyl(C₈₋₂₀)amidoalkyl(C₆₋₈)-bétaïnes ou les alkyl(C₈₋₂₀)amidoalkyl(C₆₋₈)sulfobétaïnes.
Parmi les dérivés d'amines, on peut citer les produits commercialisés sous la dénomination MIRANOL®, tels que décrits dans les brevets US 2 528 378 et US 2 781 354 et classés dans le dictionnaire CTFA, 3^{ème} édition, 1982, sous les dénominations Amphocarboxy-glycinate et Amphocarboxypropionate de structures respectives (1) et (2) :

Rₐ-CONHCH₂CH₂-N(R_{b})(R_{c})(CH₂COO-) (1)

dans laquelle :
Rₐ représente un groupe alkyle dérivé d'un acide Rₐ-COOH présent dans l'huile de coprah hydrolysée, un groupe heptyle, nonyle ou undécyle,
R_{b} représente un groupe bêta-hydroxyéthyle, et
R_{c} représente un groupe carboxyméthyle ;
et

Rₐ'-CONHCH₂CH₂-N(B)(C) (2)

dans laquelle :
B représente -CH₂CH₂OX',
C représente -(CH₂)_{z}-Y', avec z = 1 ou 2,
X' représente le groupe -CH₂CH₂-COOH ou un atome d'hydrogène,
Y' représente -COOH ou le groupe -CH₂-CHOH-SO₃H,
Rₐ' représente un groupe alkyle d'un acide Rₐ'-COOH présent dans l'huile de coprah ou dans l'huile de lin hydrolysée, un groupe alkyle, notamment en C₁₇ et sa forme iso, un groupe en C₁₇ insaturé.
Ces composés sont classés dans le dictionnaire CTFA, 5^{ème} édition, 1993, sous les dénominations cocoamphodiacétate de disodium, lauroamphodiacétate de disodium, caprylamphodiacétate de disodium, capryloamphodiacétate de disodium, cocoamphodipropionate de disodium, lauroamphodipropionate de disodium, caprylamphodipropionate de disodium, capryloamphodipropionate de disodium, acide lauroamphodipropionique, acide cocoamphodipropionique.
A titre d'exemple, on peut citer le cocoamphodiacétate commercialisé par la société RHODIA sous la dénomination commerciale MIRANOL® C2M concentré.
Parmi les tensioactifs amphotères, on utilise de préférence les (alkyle en C₈₋₂₀)-bétaïnes, les (alkyle en C₈₋₂₀)-amido(alkyle en C₆₋₈)bétaïnes, les alkylamphodiacétates et leurs mélanges.

Les tensioactifs non-ioniques utilisables dans les compositions de la présente invention sont des composés bien connus en soi (voir notamment à cet égard "Handbook of Surfactants" par M.R. PORTER, éditions Blackie & Son (Glasgow and London), 1991, pp 116-178). Ils sont choisis notamment parmi les alcools, les alpha-diols, les alkyl(C₁₋₂₀)phénols ou les acides gras polyéthoxylés, polypropoxylés ou polyglycérolés, ayant une chaîne grasse comportant, par exemple, de 8 à 18 atomes de carbone, le nombre de groupements oxyde d'éthylène ou oxyde de propylène pouvant aller notamment de 2 à 50 et le nombre de groupements glycérol pouvant aller notamment de 2 à 30.

On peut également citer les condensats d'oxyde d'éthylène et d'oxyde de propylène sur des alcools gras ; les amides gras polyéthoxylés ayant de préférence de 2 à 30 motifs d'oxyde d'éthylène, les amides gras polyglycérolés comportant en moyenne de 1 à 5 groupements glycérol et en particulier de 1,5 à 4, les esters d'acides gras du sorbitane éthoxylés ayant de 2 à 30 motifs d'oxyde d'éthylène, les esters d'acides gras du saccharose, les esters d'acides gras du polyéthylèneglycol, les (alkyle en C₆₋₂₄)polyglycosides, les dérivés de N-(alkyle en C₆₋₂₄)glucamine, les oxydes d'amines tels que les oxydes d'(alkyle en C₁₀₋₁₄)amines ou les oxydes de N-(acyle en C₁₀₋₁₄)-aminopropylmorpholine.
Parmi les tensioactifs non-ioniques cités ci-dessus, on utilise de préférence les (alkyle en C₆₋₂₄)polyglycosides.

La quantité d'agents tensioactifs anioniques est de préférence comprise entre 3 % et 35 % en poids, en particulier entre 5 % et 25 % en poids, rapportée au poids total de la composition cosmétique.

La quantité totale d'agents tensioactifs amphotères et/ou non ioniques, est de préférence comprise entre 0,5 et 30 %, et en particulier entre 1 et 20 % rapportée au poids total de la composition.

Le pH des compositions de la présente invention est de préférence compris entre 2 et 11, et en particulier entre 3 et 10.

Le milieu liquide des compositions de l'invention est aqueux ou hydroalcoolique, c'est-à-dire que, dans ce dernier cas, les compositions contiennent en plus d'une phase aqueuse, un ou plusieurs solvants choisis parmi les alcools inférieurs tels que l'éthanol ou l'isopropanol, et les polyols tels que le glycérol, le propylèneglycol et les polyéthylèneglycols.
Les compositions selon l'invention peuvent également contenir des principes actifs cosmétiques ou des additifs de formulation tels que des polymères filmogènes anioniques, non-ioniques, cationiques ou amphotères, des épaississants polymériques naturels ou synthétiques, anioniques, amphotères, zwittérioniques, non ioniques ou cationiques, associatifs ou non, des épaississants non polymériques comme des acides ou des électrolytes, des agents tensioactifs cationiques, des agents nacrants, des agents opacifiants, des colorants ou pigments, des parfums, des huiles minérales, végétales et/ou synthétiques, des cires dont les céramides, des vitamines, des filtres UV, des agents antipelliculaires, des agents anti-radicalaires, des agents plastifiants, des agents conservateurs ou des agents de stabilisation du pH.
L'homme de métier veillera à choisir les éventuels additifs et leur quantité de manière à ce qu'ils ne nuisent pas aux propriétés intéressantes des compositions de lavage des fibres kératiniques de la présente invention.

L'invention est illustrée à l'aide de l'exemple suivant.

### Exemple 1

On prépare les trois shampooings suivants (pourcentages en matières actives) :

| | A (selon l'invention) | B (comparatif) | C (comparatif) |
|---|---|---|---|
| agent tensioactif anionique^{(a)} | 17 % | 17 % | 17% |
| agent tensioactif amphotère^{(b)} | 2,5 % | 2,5 % | 2,5 % |
| copolymère diblocs PS-b-POE^{(c)} | 0,5 % | - | - |
| copolymère diblocs PEO-b-PPO^{(d)} | - | - | 0,5 % |
| amodiméthicone^{(e)} | 1 % | 1 % | 1 % |
| eau | q.s.p. 100 % | q.s.p. 100 % | q.s.p. 100 % |

| | | | |
|---|---|---|---|
| (a) lauryléthersulfate de sodium (2 OE) | | | |
| (b) cocoyibétaïne | | | |
| (c) copolymère diséquencé poly(styrène-b-oxyde d'éthylène) commercialisé sous la dénomination Tegomer® SE 1010 par la société GOLDSCHMIT | | | |
| (d) copolymère séquencé poly(oxyde d'éthylène-b-oxyde de propylène) commercialisé sous la dénomination Synperoic PE/F 127 par la société ICI | | | |
| (e) DC 939, Dow Chemical | | | |

Chacun de ces shampooings est testé sur une mèche de cheveux sensibilisés ayant une solubilité alcaline de 20 %. Chaque mèche est évaluée, après séchage, par un groupe de dix experts.
Dix experts sur dix trouvent que la mèche lavée avec le shampooing de l'invention (Composition A) et séchée est plus enrobée, plus lisse et plus glissante que celle lavée avec la Composition B (comparative) ne contenant pas de polymère séquencé diblocs mais uniquement une silicone.
Neuf experts sur dix ont trouvé que la mèche lavée avec le shampooing de l'invention (Composition A) et séchée est plus douce, plus glissante et plus lisse que celle lavée avec la Composition C contenant une silicone en association avec un copolymère diséquencé non-ionique de l'art antérieur.

## Revendications

1. Composition de lavage des matières kératiniques, de préférence des fibres kératiniques, contenant, dans un milieu aqueux ou hydro-alcoolique cosmétiquement acceptable,
• au moins un copolymère linéaire séquencé amphiphile, anionique ou non-ionique, comprenant au moins un bloc hydrophobe et au moins un bloc hydrophile, à l'exclusion des copolymères séquencés d'oxyde d'éthylène et d'oxyde de propylène, des copolymères séquencés à motifs uréthanne et des copolymères séquencés à motifs siloxane,
• au moins une silicone non-volatile à température ambiante, et
• au moins un agent tensioactif anionique associé à au moins un agent tensioactif non-ionique et/ou au moins un agent tensioactif amphotère.

2. Composition de lavage selon la revendication 1, **caractérisée par le fait que** le copolymère linéaire séquencé amphiphile est dissous ou dispersé dans le milieu aqueux ou hydroalcoolique.

3. Composition de lavage selon la revendication 2, **caractérisée par le fait que** le copolymère linéaire séquencé amphiphile est soluble dans l'eau.

4. Composition de lavage selon l'une des revendications précédentes, **caractérisée par le fait que** le ou les blocs hydrophiles du copolymère linéaire séquencé amphiphile sont formés de monomères hydrosolubles choisis parmi les monomères hydrosolubles anioniques, les monomères hydrosolubles non-ioniques ou un mélange de ceux-ci.

5. Composition de lavage selon la revendication 4, **caractérisée par le fait que** les monomères hydrosolubles anioniques sont choisis parmi les acides carboxyliques à insaturation éthylénique, l'acide 2-acrylamido-2-méthylpropanesulfonique, l'acide styrènesulfonique, l'acide vinylsulfonique et l'acide vinylphosphonique.

6. Composition de lavage selon la revendication 4, **caractérisée par le fait que** les monomères hydrosolubles non-ioniques sont choisis parmi l'acrylamide, les acrylamides N-alkylés en C₁₋₆ ou N,N-dialkylés en C₁₋₃, l'acrylate de polyéthylèneglycol, le méthacrylate de polyéthylèneglycol, le N-vinylacétamide, le N-méthyl-N-vinylacétamide, le N-vinylformamide, le N-méthyl-N-vinylformamide, les N-vinyllactames comportant un groupe cyclique de 4 à 9 atomes de carbone, l'alcool vinylique, l'oxyde d'éthylène, l'acrylate d'hydroxyéthyle, l'acrylate d'hydroxypropyle, le méthacrylate d'hydroxyéthyle et le méthacrylate d'hydroxypropyle.

7. Composition de lavage selon l'une quelconque des revendications précédentes, **caractérisée par le fait que** le ou les blocs hydrophobes sont formés de monomères insolubles dans l'eau choisis parmi les monomères vinylaromatiques, les diènes et les dérivés alkylés des diènes, le chloroprène, les acrylates d'alkyle en C₁₋₁₀, d'aryle en C₆₋₁₀ ou d'aralkyle en C₁₋₁₀, les méthacrylates d'alkyle en C₁₋₁₀, d'aryle en C₆₋₁₀ ou d'aralkyle en C₁₋₁₀, l'acétate de vinyle, les vinyléthers de formule CH₂=CH-O-R et les allyléthers de formule CH₂=CH-CH₂-O-R où R représente un groupe alkyle en C₁₋₆, l'acrylonitrile, le chlorure de vinyle, le chlorure de vinylidène, la caprolactone, l'éthylène, le propylène et les monomères vinyliques fluorés ou à chaîne perfluorée.

8. Composition de lavage selon l'une quelconque des revendications précédentes, **caractérisée par le fait que** le ou les blocs hydrophiles contiennent jusqu'à 25 % en moles, de préférence jusqu'à 10 % en moles, et idéalement jusqu'à 5 % en moles, d'un ou de plusieurs monomères insolubles dans l'eau selon la revendication 7.

9. Composition de lavage selon l'une quelconque des revendications précédentes, **caractérisée par le fait que** le ou les blocs hydrophobes contiennent jusqu'à 25 % en moles, de préférence jusqu'à 10 % en moles, et idéalement jusqu'à 5 % en moles, d'un ou de plusieurs monomères hydrosolubles selon l'une quelconque des revendications 4 à 6.

10. Composition de lavage selon l'une quelconque des revendications précédentes, **caractérisée par le fait que** le ou les copolymères séquencés amphiphile sont présents en une concentration allant de 0,01 à 20 %, de préférence de 0,1 à 5 %, rapportée au poids total de la composition de lavage.

11. Composition de lavage selon l'une quelconque des revendications précédentes, **caractérisée par le fait que** la ou les silicones non-volatiles sont choisies parmi les huiles, les cires, les résines et les gommes de silicone, réticulées ou non-réticulées, organomodifiées ou non.

12. Composition de lavage selon l'une quelconque des revendications précédentes, **caractérisée par le fait que** la ou les silicones non-volatiles sont présentes à une concentration comprise entre 0,01 et 20 %, rapportée au poids total de la composition de lavage.

13. Composition de lavage selon l'une quelconque des revendications précédentes, **caractérisée par le fait que** le ou les agents tensioactifs anioniques sont choisis parmi les alkylsulfates, les alkyléthersulfates et les alkyléthercarboxylates, et leurs mélanges, en particulier sous forme de sels de métaux alcalins ou alcalino-terreux, d'ammonium, d'amine ou d'aminoalcool.

14. Composition de lavage selon l'une quelconque des revendications précédentes, **caractérisée par le fait que** la concentration d'agents tensioactifs anioniques est comprise entre 3 et 35 % en poids, de préférence entre 5 et 25 % en poids, rapportée au poids total de la composition.

15. Composition de lavage selon l'une quelconque des revendications précédentes, **caractérisée par le fait que** l'agent tensioactif non-ionique est un (alkyle en C₆₋₂₄)-polyglycoside.

16. Composition de lavage selon l'une quelconque des revendications précédentes, **caractérisée par le fait que** l'agent tensioactif amphotère est choisi parmi les (alkyle en C₈₋₂₀)-bétaïnes, les (alkyle en C₈₋₂₀)-amido(alkyle en C₆₋₈)bétaïnes, les alkylamphodiacétates et leurs mélanges.

17. Composition de lavage selon l'une quelconque des revendications précédentes, **caractérisée par le fait que** la quantité totale d'agents tensioactifs amphotères et/ou non ioniques est comprise entre 0,5 et 30 %, et en particulier entre 1 et 20 %, rapportée au poids total de la composition.

18. Utilisation d'une composition selon l'une quelconque des revendications précédentes pour le lavage des fibres kératiniques.

## Claims

1. Composition for washing keratinous substances, preferably keratinous fibres, comprising, in a cosmetically acceptable aqueous or aqueous/alcoholic medium,
• at least one anionic or nonionic, amphiphilic linear block copolymer comprising at least one hydrophobic block and at least one hydrophilic block, with the exception of block copolymers of ethylene oxide and of propylene oxide, block copolymers comprising urethane units and block copolymers comprising siloxane units,
• at least one silicone which is nonvolatile at ambient temperature, and
• at least one anionic surface-active agent in combination with at least one nonionic surface-active agent and/or at least one amphoteric surface-active agent.

2. Washing composition according to Claim 1, **characterized in that** the amphiphilic linear block copolymer is dissolved or dispersed in the aqueous or aqueous/alcoholic medium.

3. Washing composition according to Claim 2, **characterized in that** the amphiphilic linear block copolymer is soluble in water.

4. Washing composition according to one of the preceding claims, **characterized in that** the hydrophilic block or blocks of the amphiphilic linear block copolymer are formed of water-soluble monomers chosen from anionic water-soluble monomers, nonionic water-soluble monomers or a mixture of these.

5. Washing composition according to Claim 4, **characterized in that** the anionic water-soluble monomers are chosen from carboxylic acids comprising ethylenic unsaturation, 2-acrylamido-2-methylpropanesulphonic acid, styrenesulphonic acid, vinylsulphonic acid and vinylphosphonic acid.

6. Washing composition according to Claim 4, **characterized in that** the nonionic water-soluble monomers are chosen from acrylamide, N-(C₁₋₆ alkylated)acrylamides or N,N-di(C₁₋₃ alkylated)acrylamides, polyethylene glycol acrylate, polyethylene glycol methacrylate, N-vinylacetamide, N-methyl-N-vinylacetamide, N-vinylformamide, N-methyl-N-vinylformamide, N-vinyllactams comprising a cyclic group of 4 to 9 carbon atoms, vinyl alcohol, ethylene oxide, hydroxyethyl acrylate, hydroxypropyl acrylate, hydroxyethyl methacrylate and hydroxypropyl methacrylate.

7. Washing composition according to any one of the preceding claims, **characterized in that** the hydrophobic block or blocks are formed of water-insoluble monomers chosen from vinylaromatic monomers, dienes and alkylated derivatives of dienes, chloroprene, C₁₋₁₀ alkyl, C₆₋₁₀ aryl or C₁₋₁₀ aralkyl acrylates, C₁₋₁₀ alkyl, C₆₋₁₀ aryl or C₁₋₁₀ aralkyl methacrylates, vinyl acetate, vinyl ethers of formula CH₂=CH-O-R and allyl ethers of formula CH₂=CH-CH₂-O-R where R represents a C₁₋₆ alkyl group, acrylonitrile, vinyl chloride, vinylidene chloride, caprolactone, ethylene, propylene and fluorinated vinyl monomers or vinyl monomers comprising a perfluorinated chain.

8. Washing composition according to any one of the preceding claims, **characterized in that** the hydrophilic block or blocks comprise up to 25 mol%, preferably up to 10 mol% and ideally up to 5 mol% of one or more water-insoluble monomers according to Claim 7.

9. Washing composition according to any one of the preceding claims, **characterized in that** the hydrophobic block or blocks comprise up to 25 mol%, preferably up to 10 mol% and ideally up to 5 mol% of one or more water-soluble monomers according to any one of Claims 4 to 6.

10. Washing composition according to any one of the preceding claims, **characterized in that** the amphiphilic block copolymer or copolymers are present at a concentration ranging from 0.01 to 20%, preferably from 0.1 to 5%, with respect to the total weight of the washing composition.

11. Washing composition according to any one of the preceding claims, **characterized in that** the nonvolatile silicone or silicones are chosen from silicone oils, waxes, resins and gums which may or may not be crosslinked and which may or may not be organomodified.

12. Washing composition according to any one of the preceding claims, **characterized in that** the nonvolatile silicone or silicones are present at a concentration of between 0.01 and 20% with respect to the total weight of the washing composition.

13. Washing composition according to any one of the preceding claims, **characterized in that** the anionic surface-active agent or agents are chosen from alkyl sulphates, alkyl ether sulphates and alkyl ether carboxylates, and their mixtures, in particular in the form of alkali metal or alkaline earth metal, ammonium, amine or aminoalcohol salts.

14. Washing composition according to any one of the preceding claims, **characterized in that** the concentration of anionic surface-active agents is between 3 and 35% by weight, preferably between 5 and 25% by weight, with respect to the total weight of the composition.

15. Washing composition according to any one of the preceding claims, **characterized in that** the nonionic surface-active agent is a (C₆₋₂₄ alkyl)polyglycoside.

16. Washing composition according to any one of the preceding claims, **characterized in that** the amphoteric surface-active agent is chosen from (C₈₋₂₀ alkyl) betaines, (C₈₋₂₀ alkyl) amido(C₆₋₈ alkyl) betaines, alkylamphodiacetates and their mixtures.

17. Washing composition according to any one of the preceding claims, **characterized in that** the total amount of amphoteric and/or nonionic surface-active agents is between 0.5 and 30% and in particular between 1 and 20% with respect to the total weight of the composition.

18. Use of a composition according to any one of the preceding claims for washing keratinous fibres.

## Patentansprüche

1. Zusammensetzung zum Waschen von Keratinmaterialien, vorzugsweise Keratinfasern, enthaltend in einem kosmetisch unbedenklichen wäßrigen oder wäßrig-alkoholischen Medium
• mindestens ein amphiphiles, anionisches oder nichtionisches lineares Blockcopolymer mit mindestens einem hydrophoben Block und mindestens einem hydrophilen Block mit Ausnahme von Blockcopolymeren von Ethylenoxid und Propylenoxid, Blockcopolymeren mit Urethaneinheiten und Blockcopolymeren mit Siloxaneinheiten,
• mindestens ein bei Umgebungstemperatur nichtflüchtiges Silikon und
• mindestens ein anionisches Tensid in Kombination mit mindestens einem nichtionischen Tensid und/oder mindestens einem amphoteren Tensid.

2. Waschzusammensetzung nach Anspruch 1, **dadurch gekennzeichnet, daß** das amphiphile lineare Blockcopolymer in dem wäßrigen oder wäßrig-alkoholischen Medium gelöst oder dispergiert ist.

3. Waschzusammensetzung nach Anspruch 2, **dadurch gekennzeichnet, daß** das amphiphile lineare Blockcopolymer wasserlöslich ist.

4. Waschzusammensetzung nach einem der vorhergehenden Ansprüche, **dadurch gekennzeichnet, daß** der hydrophile Block bzw. die hydrophilen Blöcke des amphiphilen linearen Blockcopolymers aus wasserlöslichen Monomeren, die unter anionischen wasserlöslichen Monomeren, nichtionischen wasserlöslichen Monomeren oder einer Mischung davon ausgewählt sind, gebildet ist bzw. sind.

5. Waschzusammensetzung nach Anspruch 4, **dadurch gekennzeichnet, daß** die anionischen wasserlöslichen Monomere unter ethylenisch ungesättigten Carbonsäuren, 2-Acrylamido-2-methylpropansulfonsäure, Styrolsulfonsäure, Vinylsulfonsäure und Vinylphosphonsäure ausgewählt sind.

6. Waschzusammensetzung nach Anspruch 4, **dadurch gekennzeichnet, daß** die nichtionischen wasserlöslichen Monomere unter Acrylamid, N-C₁₋₆-Alkylacrylamiden oder N,N-Di-C₁₋₃-alkylacrylamiden, Polyethylenglykolacrylat, Polyethylenglykolmethacrylat, N-Vinylacetamid, N-Methyl-N-vinylacetamid, N-Vinylformamid, N-Methyl-N-vinylformamid, N-Vinyllactamen mit einer cyclischen Gruppe mit 4 bis 9 Kohlenstoffatomen, Vinylalkohol, Ethylenoxid, Hydroxyethylacrylat, Hydroxypropylacrylat, Hydroxyethylmethacrylat und Hydroxypropylmethacrylat ausgewählt sind.

7. Waschzusammensetzung nach einem der vorhergehenden Ansprüche, **dadurch gekennzeichnet, daß** der hydrophobe Block bzw. die hydrophoben Blöcke aus wasserunlöslichen Monomeren, die unter vinylaromatischen Monomeren, Dienen und Alkylderivaten von Dienen, Chloropren, C₁₋₁₀-Alkylacrylaten, C₆₋₁₀-Arylacrylaten oder C₁₋₁₀-Aralkylacrylaten, C₁₋₁₀-Alkylmethacrylaten, C₆₋₁₀-Arylmethacrylaten oder C₁₋₁₀-Aralkylmethacrylaten, Vinylacetat, Vinylethern der Formel CH₂=CH-O-R und Allylethern der Formel CH₂=CH-CH₂-O-R, worin R für eine C₁₋₆-Alkylgruppe steht, Acrylnitril, Vinylchlorid, Vinylidenchlorid, Caprolacton, Ethylen, Propylen und fluorierten Vinylmonomeren oder Vinylmonomeren mit perfluorierter Kette ausgewählt sind, gebildet ist bzw. sind.

8. Waschzusammensetzung nach einem der vorhergehenden Ansprüche, **dadurch gekennzeichnet, daß** der hydrophile Block bzw. die hydrophilen Blöcke bis zu 25 Mol-%, vorzugsweise bis zu 10 Mol-%, und idealerweise bis zu 5 Mol-%, eines oder mehrerer wasserunlöslicher Monomere gemäß Anspruch 7 enthält bzw. enthalten.

9. Waschzusammensetzung nach einem der vorhergehenden Ansprüche, **dadurch gekennzeichnet, daß** der hydrophobe Block bzw. die hydrophoben Blöcke bis zu 25 Mol-%, vorzugsweise bis zu 10 Mol-%, und idealerweise bis zu 5 Mol-%, eines oder mehrerer wasserlöslicher Monomere gemäß den Ansprüchen 4 bis 6 enthält bzw. enthalten.

10. Waschzusammensetzung nach einem der vorhergehenden Ansprüche, **dadurch gekennzeichnet, daß** das amphiphile Blockcopolymer bzw. die amphiphilen Blockcopolymere in einer Konzentration im Bereich von 0,01 bis 20%, vorzugsweise 0,1 bis 5%, bezogen auf das Gesamtgewicht der Waschzusammensetzung, vorliegt bzw. vorliegen.

11. Waschzusammensetzung nach einem der vorhergehenden Ansprüche, **dadurch gekennzeichnet, daß** das nichtflüchtige Silikon bzw. die nichtflüchtigen Silikone unter gegebenenfalls organisch modifizierten, vernetzten oder unvernetzten Silikonölen, -wachsen, -harzen und -gummen ausgewählt ist bzw. sind.

12. Waschzusammensetzung nach einem der vorhergehenden Ansprüche, **dadurch gekennzeichnet, daß** das nichtflüchtige Silikon bzw. die nichtflüchtigen Silikone in einer Konzentration zwischen 0,01 und 20%, bezogen auf das Gesamtgewicht der Waschzusammensetzung, vorliegt bzw. vorliegen.

13. Waschzusammensetzung nach einem der vorhergehenden Ansprüche, **dadurch gekennzeichnet, daß** das anionische Tensid bzw. die anionischen Tenside unter Alkylsulfaten, Alkylethersulfaten und Alkylethercarboxylaten und Mischungen davon, insbesondere in Form von Alkalimetall-, Erdalkalimetall-, Ammonium-, Amin- oder Aminoalkoholsalzen, ausgewählt ist bzw. sind.

14. Waschzusammensetzung nach einem der vorhergehenden Ansprüche, **dadurch gekennzeichnet, daß** die Konzentration der anionischen Tenside zwischen 3 und 35 Gew.-%, vorzugsweise zwischen 5 und 25 Gew.-%, bezogen auf das Gesamtgewicht der Zusammensetzung, liegt.

15. Waschzusammensetzung nach einem der vorhergehenden Ansprüche, **dadurch gekennzeichnet, daß** es sich bei dem nichtionischen Tensid um ein (C₆₋₂₄-Alkyl)polyglykosid handelt.

16. Waschzusammensetzung nach einem der vorhergehenden Ansprüche, **dadurch gekennzeichnet, daß** das amphotere Tensid unter (C₈₋₂₀-Alkyl)betainen, (C₈₋₂₀-Alkyl)amido(C₆₋₈-alkyl)betainen, Alkylamphodiacetaten und Mischungen davon ausgewählt ist.

17. Waschzusammensetzung nach einem der vorhergehenden Ansprüche, **dadurch gekennzeichnet, daß** die Gesamtmenge an amphoteren und/oder nichtionischen Tensiden zwischen 0,5 und 30%, vorzugsweise zwischen 1 bis 20%, bezogen auf das Gesamtgewicht der Zusammensetzung, liegt.

18. Verwendung einer Zusammensetzung nach einem der vorhergehenden Ansprüche zum Waschen von Keratinfasern.
